# EUROPEAN PATENT APPLICATION

(11) **EP 4 541 359 A1**
(43) Date of publication of application: **23.04.2025**
(21) Application number: 23827031.8
(22) Date of filing: 12.06.2023
(51) Int. Cl.: A61K 35/32, A61K 9/14, A61K 31/7105, A61K 35/28, A61K 47/46, A61K 48/00, A61P 9/00, A61P 9/08, A61P 9/10, A61P 17/00, A61P 19/02, A61P 29/00, A61P 37/02, A61P 37/06, A61P 43/00, C12N 15/113

(54) **PTX3 EXPRESSION CONTROL AGENT, PROPHYLACTIC DRUG OR THERAPEUTIC DRUG FOR VASCULITIS OR HARDENING OF SKIN ASSOCIATED WITH RHEUMATOID ARTHRITIS, AUTOIMMUNE DISEASE, METHOD FOR IMPROVING VASCULITIS OR HARDENING OF SKIN ASSOCIATED WITH RHEUMATOID ARTHRITIS, AUTOIMMUNE DISEASE**

(30) Priority: 20.06.2022 JP 2022098639; 20.05.2023 JP 2023083545
(71) Applicant: Dexon Pharmaceuticals Inc., Tokyo, 103-0027 (JP)
(72) Inventor: KOGA Shoji, Tokyo 102-0082 (JP)
(74) Representative: Godemeyer Blum Lenze Patentanwälte Partnerschaft mbB - werkpatent
(86) International application number: PCT/JP2023/021641
(87) International publication number: WO 2023/248845

(57) **Abstract**

Provided are: a novel PTX3 expression controlling agent, in which the expression controlling agent includes small extracellular vesicles, the small extracellular vesicles contain a miRNA targeting a gene involved in expression of PTX3 as a target gene, and the PTX3 expression controlling agent is capable of controlling expression of PTX3 using the small extracellular vesicle-derived miRNA; a prophylactic drug or therapeutic drug for vasculitis or skin hardening associated with rheumatoid arthritis and autoimmune diseases; and a method for improving vasculitis or skin hardening associated with rheumatoid arthritis and autoimmune diseases. The small extracellular vesicles are preferably exosomes.

## Description

### Technical Field

The present invention relates to a PTX3 expression controlling agent; a prophylactic drug or therapeutic drug for vasculitis or skin hardening associated with rheumatoid arthritis and autoimmune diseases; and a method for improving vasculitis or skin hardening associated with rheumatoid arthritis and autoimmune diseases.

### Background Art

Pentraxin 3 (PTX3) is also referred to as TSG-14 (TNF-inducible gene 14 protein). PTX3 is a member of the pentraxin superfamily consisting of a group of evolutionarily conserved proteins characterized by a pentraxin domain, which is a structural motif. PTX3 is produced by a variety of cell types such as endothelial cells, smooth muscle cells, adipocytes, fibroblasts, mononuclear phagocytes, and dendritic cells. The expression of PTX3 is induced by IL-1, TNF (Tumor necrosis factor), and primary inflammatory signals of microorganisms and the like. PTX3 is an acute phase glycoprotein and plays a role in the regulation of innate immune resistance to pathogens, inflammation, tissue remodeling and repair, female fertility, and cancer. Furthermore, pentraxin 3 is a biomarker for cardiovascular diseases, and in patients with cardiovascular diseases, an increase in the concentration of plasma pentraxin 3 is recognized.

In recent years, it has become known that PTX3 is involved in rheumatoid arthritis (NPL 1). NPL 1 describes that the level of PTX3 increases in the synovial fluid of rheumatoid arthritis joints, and that the progression of rheumatoid arthritis can be suppressed by suppressing PTX3.

Furthermore, it is known that PTX3 blocks vasculitis associated with autoimmune diseases (NPL 2).

On the other hand, although research on microRNA (miRNA) is in progress, not much is known about the functions of miRNAs involved in PTX3 and the target genes.

### Citation List

### Non Patent Literature

NPL 1: Int. J. Med. Sci., (2021) 18(8): 1886-1898
NPL 2: Front. Immunol., (2019) 10, 1135

### Summary of Invention

### Technical Problem

NPL 1 and NPL 2 have no description on miRNAs.

An object of the present invention is to provide a novel PTX3 expression controlling agent that can control the expression of PTX3 using miRNA derived from small extracellular vesicles (sEVs).

### Solution to Problem

The inventors of the present invention found that small extracellular vesicles containing specific microRNA can control (suppress, inhibit, or the like) the expression of PTX3 protein and/or PTX3 gene (Ptx3).

Specifically, the present invention and preferred configurations of the present invention are as follows.

[1] A PTX3 expression controlling agent,
   wherein the expression controlling agent includes small extracellular vesicles, and
   the small extracellular vesicles contain a miRNA targeting a gene involved in expression of PTX3 as a target gene.
[2] The PTX3 expression controlling agent according to [1], wherein the small extracellular vesicles are exosomes.
[3] The PTX3 expression controlling agent according to [1], wherein the expression controlling agent includes at least one type of hsa-miR-224-5p, hsa-miR-374c-5p, and hsa-miR-655-3p as the miRNA targeting a gene involved in the expression of PTX3 as a target gene.
[4] The PTX3 expression controlling agent according to [1], wherein the miRNA targeting a gene involved in the expression of PTX3 as a target gene includes at least hsa-miR-224-5p.
[5] The PTX3 expression controlling agent according to [1], wherein the miRNA targeting a gene involved in the expression of PTX3 as a target gene includes hsa-miR-224-5p and hsa-miR-655-3p.
[6] The PTX3 expression controlling agent according to [1], wherein the small extracellular vesicles contain a miRNA targeting a gene involved in the expression of PTX3 as a target gene, at a concentration higher than that in a culture supernatant of dental pulp-derived stem cells.
[7] The PTX3 expression controlling agent according to [1], wherein the small extracellular vesicles are small extracellular vesicles purified and isolated from a culture supernatant of dental pulp-derived stem cells, and the small extracellular vesicles do not contain any component other than exosomes from the culture supernatant of dental pulp-derived stem cells.
[8] A prophylactic drug or therapeutic drug for vasculitis or skin hardening associated with rheumatoid arthritis and autoimmune diseases, the prophylactic drug or therapeutic drug containing the PTX3 expression controlling agent according to [1] as an active ingredient.
[9] The prophylactic drug or therapeutic drug for vasculitis or skin hardening associated with rheumatoid arthritis and autoimmune diseases according to [8], wherein the prophylactic drug or therapeutic drug is a prophylactic drug or therapeutic drug for skin hardening and is a skin application agent.
[10] A method for improving vasculitis or skin hardening associated with rheumatoid arthritis and autoimmune diseases, the method including administering an effective amount of the PTX3 expression controlling agent according to [1] or an effective amount of the prophylactic drug or therapeutic drug for vasculitis or skin hardening associated with rheumatoid arthritis and autoimmune diseases according to [8], to a subject who has developed vasculitis or skin hardening associated with rheumatoid arthritis and autoimmune diseases.

### Advantageous Effects of Invention

According to the present invention, a novel PTX3 expression controlling agent that can control the expression of PTX3 using a small extracellular vesicle-derived miRNA, can be provided.

### Brief Description of Drawings

[FIG. 1] FIG. 1 is a heat map showing the expression levels of miRNAs expressed in exosomes (expression controlling agent of Example 1) purified from a culture supernatant of dental pulp-derived stem cells targeting a gene involved in the expression of PTX3.
[FIG. 2] FIG. 2 is a heat map showing the expression levels of miRNAs expressed in exosomes (expression controlling agent of Example 1) purified from a culture supernatant of dental pulp-derived stem cells targeting a gene involved in the expression of TSP1.
[FIG. 3] FIG. 3 is a graph showing the expression level of PTX3 gene and the expression level of TSP-1 gene quantified in each example.

### Description of Embodiments

The present invention will be described in detail below. The description of constituent requirements described below may be based on representative embodiments and specific examples; however, the present invention is not limited to such embodiments. Incidentally, a numerical value range indicated using the term "to" in the present specification means a range including the numerical values described before and after the term "to" as the lower limit value and the upper limit value.

### [PTX3 expression controlling agent]

In the PTX3 expression controlling agent of the present invention, the expression controlling agent includes small extracellular vesicles, and the small extracellular vesicles contain a miRNA targeting a gene involved in the expression of PTX3 as a target gene.

The PTX3 expression controlling agent of the present invention can control the expression of PTX3 using a small extracellular vesicle-derived miRNA. As a result, it is preferable that the PTX3 expression controlling agent of the present invention can improve vasculitis or skin hardening associated with rheumatoid arthritis and autoimmune diseases, and it is more preferable that the expression controlling agent can prevent or treat vasculitis or skin hardening.

Hereinafter, preferred embodiments of the PTX3 expression controlling agent of the present invention will be described. Incidentally, when there is no separate description of preferred embodiments of a TSP-1 expression controlling agent, the description of preferred embodiments of the PTX3 expression controlling agent of the present invention is also applicable to the description of preferred embodiments of the TSP-1 expression controlling agent.

### <Vasculitis associated with rheumatoid arthritis and autoimmune diseases>

First, a case in which the PTX3 expression controlling agent of the present invention is used for improving vasculitis associated with rheumatoid arthritis and autoimmune diseases will be described.

In the present invention, the term vasculitis is a generic name for diseases in which inflammation is observed in the blood vessels themselves, and diseases in which bleeding, blood flow disorder, and infarction occur as a result, leading to organ disorders. Vasculitis is also referred to as vasculitis syndrome or systemic vasculitis.

### (Classification according to CHCC 2012)

Vasculitis may be classified according to the blood vessel diameter of the blood vessel in which main inflammation has occurred. Classification based on the blood vessel diameter is generally carried out according to the following CHCC (Chapel Hill Consensus Conference) 2012. Among these vasculitides, the PTX3 expression controlling agent of the present invention is used to improve vasculitides associated with rheumatoid arthritis and various autoimmune diseases included in "VI. Vasculitis associated with systemic diseases".

Regarding the vasculitis associated with various autoimmune diseases, the PTX3 expression controlling agent is used to improve Takayasu's arteritis included in "I. Large vessel vasculitis", microscopic polyangiitis (MPA) and immune complex-mediated small vessel vasculitis (cryoglobulinemic vasculitis and the like), which are vasculitides associated with autoimmune diseases included in "III. Small vessel vasculitis", Behcet's diseases included in "IV. Vasculitis affecting various blood vessels", and vasculitis associated with autoimmune diseases (other than rheumatoid arthritis) included in "VI. Vasculitis associated with systemic diseases".

### I. Large vessel vasculitis

(I-1) Takayasu's arteritis: This is granulomatous vasculitis in many cases, and affects the aorta and its main branches. The main symptoms include systemic inflammation, pain due to vasculitis, and stenosis, blockage, and dilation of blood vessels, and even after the inflammation subsides, various organ disorders and aneurysms due to blood flow disorder become problems. The onset mechanism is presumed to be genetic in background, with environmental factors such as infection triggering the destruction of elastic arteries, mainly the aorta, by an autoimmune mechanism. The pathological characteristics of Takayasu's arteritis is an erosion image of the medial elastic fibers beginning from the adventitia side. For infiltrating cells, CD4 positive T cells, CD8 positive T cells, macrophages, NK cells, γδT cells, and the like are recognized, and some of the macrophages are observed as multinucleated giant cells that phagocytose fragmented elastic fibers. It is speculated that these cells damage the vascular wall in association with cytokine abnormality. Steroids, immunosuppressants, biological preparations (tocilizumab, TNF inhibitors, and the like), antiplatelet drugs, and the like are used as therapeutic drugs.
(I-2) Giant cell vasculitis: This is granulomatous vasculitis in many cases, and affects the aorta and its main branches. The carotid arteries, branches of the vertebral arteries, and the temporal arteries are often affected.

### II. Medium vessel vasculitis (often accompanied by inflammatory aneurysm and stenosis)

(II-1) Polyarteritis nodosa (PAN): This is necrotizing small and medium vessel vasculitis that does not cause glomerulonephritis, arteriolitis, capillaritis, or venulitis. This is not associated with antineutrophil cytoplasmic antibodies (ANCA).
(II-2) Kawasaki disease: This is vasculitis associated with mucocutaneous lymph node syndrome. It mainly affects medium and small blood vessels. The coronary arteries are highly frequently affected. The aorta and large blood vessels may also be affected.

### III. Small vessel vasculitis

(III-1) ANCA-associated vasculitis: This includes microscopic polyangiitis (MPA), granulomatosis with polyangiitis (GPA) (formerly Wegener's granulomatosis), and eosinophilic granulomatosis with polyangiitis (EGPA) (formerly Churg-Strauss syndrome).

Microscopic polyangiitis (MPA) is necrotizing vasculitis that primarily affects small blood vessels, and immune complex deposition hardly occurs. Main symptoms include rapidly progressive glomerulonephritis, pulmonary hemorrhage, interstitial pneumonia, and symptoms in organs other than kidneys and lungs (purpura, subcutaneous hemorrhage, gastrointestinal bleeding, mononeuritis multiplex, fever, weight loss, and fatigue). As the onset mechanism, it is speculated that an autoimmune mechanism is involved, in which genetic factors and environmental factors affect each other to produce autoantibody ANCA, which in turn causes vascular endothelial disorder. The pathology of vascular endothelial cell disorder caused by MPA is thought to involve inflammatory cytokines such as TNF-α, ANCA, MPO, and neutrophil activation. It is speculated that ANCA produced by an autoimmune mechanism excessively activates neutrophils together with inflammatory cytokines and releases microfibrillar networks (chromatin fibers) called neutrophil extracellular traps that contain proteolytic enzymes within neutrophils, damaging the vascular endothelium. The main treatment strategies include glucocorticoids (GCs) and immunosuppressants (including rituximab).

(III-2) Immune complex-mediated small vessel vasculitis: This includes anti-glomerular basement membrane antibody disease (anti-GBM disease), cryoglobulinemic vasculitis, IgA vasculitis (formerly Henoch-Schonlein purpura), and hypocomplementemic urticarial vasculitis (anti-C1q vasculitis).

A group of patients exhibiting immune complex-mediated vascular inflammation symptoms containing cryoglobulinemic vasculitis (CG), is referred to as cryoglobulinemic vasculitis. Cryoglobulinemia is frequently recognized in autoimmune diseases.

### IV. Vasculitis affecting various blood vessels

(IV-1) Behcet's disease: This is a vasculitis occurring in patients with Behcet's disease (characterized by recurrent stomatitis and genital ulcers, and accompanied by inflammatory lesions of the skin, eyes, joints, gastrointestinal tract, and central nervous system), and affects arteries and veins. Small vessel vasculitis, thrombotic vasculitis, thrombosis, arteritis, and aneurysms occur.

Both autoimmune mechanism and auto-inflammatory mechanism contribute to the pathology.

(IV-2) Cogan's syndrome: This is vasculitis occurring in patients with Cogan's syndrome (characterized by inflammatory eye lesions such as interstitial keratitis, uveitis, and episcleritis, and inner ear diseases such as sensorineural hearing loss and vestibular dysfunction). Arteritis (small, medium, and large vessels), aortitis, aortic aneurysm, aortic valvulitis, and mitral valvulitis occur.

### V. Vasculitis affecting single organ

This includes cutaneous leukocytoclastic angiitis; cutaneous arteritis; cutaneous small vessel vasculitis; primary central nervous system vasculitis; isolated aortitis; and testicular arteritis.

### VI. Vasculitis associated with systemic diseases

This includes Lupus vasculitis; rheumatoid vasculitis; sarcoid vasculitis; and the like. It includes vasculitis associated with autoimmune diseases (autoimmune diseases associated with vasculitis).

(VI-1) Rheumatic angiitis (RA) includes rheumatoid vasculitis (RV) of medium and small vessel vasculitis. The onset mechanism of rheumatic vasculitis is unknown; however, possible mechanisms include involvement of autoantibodies targeting blood vessels, inflammation caused by deposition of immune complexes, and activation of local cellular immunity. Deposition of immunoglobulins, C3, and C4 occurs at the inflammation sites of vasculitis. Since high rheumatoid factor levels in serum, positive immune complexes, and low complement levels, and the like are observed in RV, it is believed that there are more severe immunological abnormalities than RA. The treatment strategies include steroids, steroid pulse therapy, methotrexate (MTX), azathioprine (AZA), cyclophosphamide (CY), TNF inhibitors, tocilizumab (TCZ), rituximab (RTX), and abatacept.

(VI-2) Vasculitis associated with autoimmune diseases include Buerger's disease and the like. Buerger's disease is a segmental lesion of the limb arteries and veins, which is commonly seen in men in their 20's to 40's. Possible onset mechanisms include smoking, infection, nutritional disorders, autoimmunity, and the like.

### VII. Vasculitis associated with known causes

This includes hepatitis C virus (HCV)-associated cryoglobulinemic vasculitis; hepatitis B virus (HBV)-associated vasculitis; syphilis-associated aortitis; drug-associated immune complex-mediated vasculitis; drug-associated ANCA-associated vasculitis; cancer-associated vasculitis; hydralazine-associated microscopic polyangiitis; and the like.

### <Skin hardening>

Next, a case of using the PTX3 expression controlling agent of the present invention to improving skin hardening will be described.

Skin hardening means that the occurrence of fibrosis of skin, the occurrence of hardening of the dermis, and other types of scleroderma. Incidentally, the other types of scleroderma include systemic scleroderma and localized scleroderma.

Skin fibrosis refers to a phenomenon in which substances called extracellular matrix such as collagen fibers (collagen) increase in the skin and the internal organs, and as a result, the skin and the internal organs become hard.

Hardening of the dermis generally means that the dermis becomes hard. For example, hardening of the dermis proceeds along with aging. Hardening of the dermis due to aging occurs as a result of atrophy of the body surface blood vessels, and the like. Hardening of the dermis associated with aging induces aging-related appearance change in epidermal stem cells, such as weakening of hemidesmosomes, abnormality of division axes, and premature differentiation. **In** contrast, when the body surface blood vessels are artificially induced, the dermis of aged skin is softened, and hardness of tissues dependent on blood vessels can be improved. Furthermore, aging-related appearance change in epidermal stem cells can also be improved.

Systemic scleroderma occurs due to complex causes such as immune abnormalities (autoimmune diseases), fibrosis, and vascular abnormalities. **In** systemic scleroderma, skin hardening begins from the fingers and continuously progresses from the back of the hand to the forearm, toward the proximal side (side closer to the body).

Localized scleroderma is a disease in which sharply demarcated hardening of the skin appears in localized areas, and is completely different from systemic scleroderma. Localized scleroderma is an autoimmune disease caused by an autoimmune response to somatic mosaicism.

It is preferable that the PTX3 expression controlling agent of the present invention is used for improving at least hardening of the dermis among these types of skin hardening. PTX3 is a factor which has an action of suppressing angiogenesis, and whose expression increases along with aging. By controlling the expression of PTX3, atrophy and involution of the body surface blood vessels accompanied by aging are alleviated, the dermis can be softened, and skin hardening can be improved. In addition, since the PTX3 expression controlling agent of the present invention can improve autoimmune diseases, the PTX3 expression controlling agent can also improve systemic scleroderma and localized scleroderma.

### <Small extracellular vesicles>

The PTX3 expression controlling agent of the present invention includes small extracellular vesicles.

In the present invention, the small extracellular vesicles contain miRNA targeting a gene involved in the expression of PTX3 as a target gene.

The small extracellular vesicles are derived from dental pulp-derived stem cells or the like by, for example, secretion, budding, dispersion, or the like from mesenchymal stem cells such as dental pulp-derived stem cells, and are leached, released, or shed into the cell culture medium. It is preferable that the small extracellular vesicles are included in the culture supernatant of dental pulp-derived stem cells or the like, and it is more preferable that the small extracellular vesicles are small extracellular vesicles derived from a culture supernatant of dental pulp-derived stem cells. However, the small extracellular vesicles derived from a culture supernatant of dental pulp-derived stem cells do not necessarily have to be obtained from a culture supernatant of dental pulp-derived stem cells. For example, even when small extracellular vesicles inside dental pulp-derived stem cells are isolated by any method, as long as the small extracellular vesicles are the same as small extracellular vesicles that can be isolated from a culture supernatant of dental pulp-derived stem cells, it can be said that the small extracellular vesicles are small extracellular vesicles derived from a culture supernatant of dental pulp-derived stem cells.

The small extracellular vesicles derived from a culture supernatant of dental pulp-derived stem cells or the like may be used in a state of being included in the culture supernatant, or may be used in a state of being purified from the culture supernatant. It is preferable that the small extracellular vesicles are small extracellular vesicles purified from a culture supernatant.

The origin of the small extracellular vesicles can be determined by a known method. For example, which stem cells the small extracellular vesicles originate from among dental pulp-derived stem cells, adipose-derived stem cells, bone marrow-derived stem cells, and umbilical cord-derived stem cells, can be determined by the method described in J Stem Cell Res Ther (2018) 8:2. Specifically, the origin of each small extracellular vesicle can be determined on the basis of the miRNA pattern of the small extracellular vesicle.

### (miRNA)

In the present invention, the small extracellular vesicles contain a miRNA targeting a gene involved in the expression of PTX3 as a target gene.

In the present invention, the miRNA (microRNAs) is, for example, an RNA molecule of 21 to 25 bases (nucleotides). The miRNA can regulate gene expression by degradation of the target gene (target) mRNA or by suppression in the decoding stage.

In the present invention, the miRNA may be, for example, single-stranded (monomer) or double-stranded (dimer). Furthermore, according to the present invention, the miRNA is preferably a mature type miRNA cleaved by a ribonuclease such as Dicer.

Incidentally, the sequences of the miRNAs described in the present specification, such as hsa-miR-224-5p, are registered in known databases (for example, miRBase database) in association with accession numbers, and a person skilled in the art can unambiguously determine the sequences. For example, the accession number of hsa-miR-224-5p is MIMAT0000281, and the sequence is registered in the miRBase database. Hereinafter, the accession number of each miRNA is omitted.

However, the miRNA in the present specification also includes mutants that differ from mature miRNA such as hsa-miR-224-5p by about 1 to 5 bases. Furthermore, each miRNA in the present specification includes a polynucleotide having a base sequence that has identity with the base sequence of each miRNA (for example, hsa-miR-224-5p), or a polynucleotide having a base sequence complimentary thereto and having the function of the miRNA according to the present invention. The term "identity" refers to the degree of identicalness when the sequences to be compared are properly aligned, and means the occurrence ratio of exact amino acid matches between the sequences. The alignment can be performed by, for example, utilizing any algorithm such as BLAST. The identity is, for example, 80%, 85%, 90%, 91%, 92%, 93%, 94%, 95%, 96%, 97%, 98%, or about 99%. A polynucleotide having a base sequence having identity may have, for example, point mutation, deletion, and/or addition in the base sequence of the miRNA. The number of bases in the point mutation and the like is, for example, 1 to 5, 1 to 3, 1 or 2, or 1. Furthermore, a polynucleotide having a complimentary base sequence is a polynucleotide that hybridizes under stringent conditions with a polynucleotide having the base sequence of a miRNA, and includes a polynucleotide having the function of the miRNA according to the present invention. The stringent conditions are not particularly limited; however, for example, the conditions described in [0028] of JP2017-184642A may be mentioned, the content of which is incorporated herein by reference.

In the present invention, it is preferable that the small extracellular vesicles contain a miRNA targeting a gene involved in the expression of PTX3 as a target gene, at a concentration higher than that in a culture supernatant of dental pulp-derived stem cells. Furthermore, it is preferable that the small extracellular vesicles contain a miRNA targeting a gene involved in the expression of TSP-1 as a target gene, at a concentration higher than that in a culture supernatant of dental pulp-derived stem cells. Hereinafter, preferred embodiments of the miRNA contained in the small extracellular vesicles will be described.

### (1) miRNA targeting gene involved in expression of PTX3 as target gene

NPL 1 (Int. J. Med. Sci., (2021) 18(8): 1886-1898) describes that the level of PTX3 increases in the synovial fluid of rheumatoid arthritis joints, and that the progression of rheumatoid arthritis can be suppressed by suppressing PTX3.

Furthermore, PTX3 can block vasculitis associated with autoimmune diseases (NPL 2: Front. Immunol., (2019) 10, 1135).

PTX3 is acute inflammatory protein and is expressed in vascular endothelial cells, macrophages, fibroblasts, smooth muscle cells, and the like, and expression thereof increases in an inflammatory stimulus-dependent manner. Therefore, PTX3 serves as a marker for ischemic heart diseases such as myocardial infarction and arteriosclerosis.

Therefore, miRNAs targeting genes involved in the expression of PTX3 as target genes (hsa-miR-224-5p, hsa-miR-374c-5p, hsa-miR-655-3p, and the like are known) may be effective as a prophylactic drug or therapeutic drug for vasculitis or skin hardening associated with rheumatoid arthritis and autoimmune diseases.

Incidentally, decreased expression of miR-224 promotes the formation of arteriorsclerotic plaque that is vulnerable in acute coronary syndrome (ACS) and remodeling of blood vessels through activation of the TGF-β/Smad pathway (J. Cell. Physiol. (2018) 234, 2537-2551).

The role of IncRNA AK087124 and miR-224-5p in the pathology of arteriosclerosis was investigated, and AK087124 is upregulated while miR-224-5p is downregulated in the plasma and plaque of arteriosclerotic mice as compared with normal mice. miR-224-5p suppresses PTEN to suppress apoptosis and inflammatory responses (Archives of Biochemistry and Biophysics, (2021) 705, 15:108916).

In the present invention, when the small extracellular vesicles contain a miRNA targeting a gene involved in the expression of PTX3 (preferably, PTX3 gene) as a target gene, the small extracellular vesicles preferably function as an expression controlling agent for a gene involved in the expression of PTX3. The small extracellular vesicles are preferably a PTX3 expression inhibitor.

In this case, in the present invention, it is preferable that the small extracellular vesicles contain at least one type of hsa-miR-224-5p, hsa-miR-374c-5p, and hsa-miR-655-3p, it is more preferable that the small extracellular vesicles contain at least hsa-miR-224-5p, it is particularly preferable that the small extracellular vesicles contain hsa-miR-224-5p and hsa-miR-655-3p, and it is more particularly preferable that the small extracellular vesicles contain all of hsa-miR-224-5p, hsa-miR-374c-5p, and hsa-miR-655-3p.

It is preferable that the small extracellular vesicles contain at least one type of miRNAs targeting genes involved in the expression of PTX3 as target genes such that the Log2Ratio of the number of lead counts obtained by analysis using IMOTAis 2.0 or more, more preferably 4.0 or more, and particularly preferably 6.0 or more. It is preferable that the small extracellular vesicles contain hsa-miR-224-5p and hsa-miR-655-3p such that the Log2Ratio of the number of lead counts obtained by analysis using IMOTA is 4.0 or more for both, and it is more preferable that the small extracellular vesicles contain hsa-miR-224-5p such that the Log2Ratio is 6.0 or more.

With regard to the small extracellular vesicles, it is preferable that the expression level of hsa-miR-224-5p is 1.1 times or more, more preferably 1.5 times or more, and particularly preferably 2 times or more, as compared with exosomes obtained from a culture supernatant of adipose-derived stem cells or exosomes obtained from a culture supernatant of umbilical cord-derived stem cells.

When the small extracellular vesicles are a PTX3 expression inhibitor, it is preferable that the expression of the gene of PTX3 in any cell can be suppressed to be 0.8-fold or less, more preferably 0.7-fold or less, and particularly preferably 0.6-fold or less, the normal level (the case of untreated cells).

### (2) miRNA targeting gene involved in expression of thrombospondin 1 as target gene

Thrombospondin 1 is widely known as an angiogenesis inhibitory factor. miR-18 and miR-19 regulate the expression of the connective tissue growth factor (CTGF) of extracellular matrix (ECM) proteins and thrombospondin 1 (TSP-1) in age-related heart failure. Aging reduces miR-18 and miR-19, and when the levels of CTGF and TSP-1 increase, cardiac disorder is likely to occur (Aging Cell, 10, 5, 769-779 (2011)).

Thrombospondin 1 (TSP1) is a target of miR-221. miR-221 significantly suppressed Nx-induced upregulation of cardiac TSP1 and p-SMAD3, reduced myocardial fibrosis (picro-sirius), and improved cardiac function (Mol. Ther. Nucleic Acids (2020) 22:803-814).

Therefore, miRNAs targeting genes involved in the expression of thrombospondin 1 (the following TSP1-related miRNA group) are effective as therapeutic drugs of vasculitis.

### TSP1-related miRNA group:

hsa-let-7a-5p, hsa-let-7b-5p, hsa-let-7c-5p, hsa-let-7d-5p, hsa-let-7e-5p,
hsa-let-7f-5p, hsa-let-7g-5p, hsa-let-7i-5p, hsa-miR-101-3p, hsa-miR-125a-3p,
hsa-miR-1273g-3p, hsa-miR-132-3p, hsa-miR-139-5p, hsa-miR-141-3p, hsa-miR-143-3p,
hsa-miR-144-3p, hsa-miR-155-5p, hsa-miR-182-5p, hsa-miR-194-5p, hsa-miR-198,
hsa-miR-19a-3p, hsa-miR-19b-3p, hsa-miR-205-5p, hsa-miR-206, hsa-miR-211-3p,
hsa-miR-212-3p, hsa-miR-221-3p, hsa-miR-222-3p, hsa-miR-27b-3p, hsa-miR-297,
hsa-miR-300, hsa-miR-30a-3p, hsa-miR-30a-5p, hsa-miR-3149, hsa-miR-331-3p,
hsa-miR-338-3p, hsa-miR-340-5p, hsa-miR-3609, hsa-miR-3665, hsa-miR-377-3p,
hsa-miR-377-5p, hsa-miR-381-3p, hsa-miR-382-5p, hsa-miR-410-3p, hsa-miR-411-3p,
hsa-miR-4257, hsa-miR-4270, hsa-miR-4450, hsa-miR-4458, hsa-miR-4495,
hsa-miR-4500, hsa-miR-455-3p, hsa-miR-4655-5p, hsa-miR-4739, hsa-miR-491-5p,
hsa-miR-493-3p, hsa-miR-493-5p, hsa-miR-5003-5p, hsa-miR-509-5p, hsa-miR-5096, and
hsa-miR-543, hsa-miR-582-5p, hsa-miR-613, hsa-miR-650, hsa-miR-657, hsa-miR-675-3p, hsa-miR-7-1-3p, hsa-miR-766-5p, and hsa-miR-98-5p.

In the TSP1-related miRNA group, in addition to the above-described miR-19 and miR-221, the following specific miRNAs are known to be able to improve vasculitis.

### (2-1) Concerning hsa-let-7a-5p, hsa-let-7b-5p, hsa-let-7f, and hsa-miR-132-3p

When let-7a and let-7b are overexpressed, they suppress oxLDL-induced endothelial cell apoptosis, NO deficiency, excessive production of active oxygen, increased expression of LOX-1, and downregulation of endothelial nitric oxide synthase (eNOS). let-7a and let-7b have protective action against endothelial cell injury caused by oxidized low-density lipoprotein (Plos one (2014) 9, (9):e106540).

Secreted exosomal miRs (miR-210, miR-23a-3p, miR-424, let-7f, miR-30b, miR-30c, miR-126, miR-21, miR-132, miR-130a-3p, miR-214, miR-378, miR-126, miR-133, and let-7b-5p) induce cardiac angiogenesis and revascularization and protect against myocardial ischemia by increasing the blood flow to the ischemic myocardium (Heart Failure Reviews, (2021) 26(1), 205-213).

Extracellular vesicles derived from human adipose-derived stem cells (hADSC-EV) promote exogenous angiogenesis and enhance the proliferation, migration, tube formation, and VEGF secretion of vascular endothelial cells. The let-7 family, which is a type of hypoxia-related microRNA, is enriched in hypoxic hADSC-EVs, which contributes to angiogenesis through the let-7/argonaute 1 (AGO1)/VEGF signaling pathway (Sci. Rep. (2020) 10(1):5313).

let-7a-5p targets IGF2BP3 and suppress death of cardiomyocytes (Int. J. Mol. Med. (2020) 45(2):451-460).

### (2-2) Concerning hsa-miR-143-3p

miR-143-3p mimic further improves the expression of TC, TG, and LDLC in the blood of rabbits with coronary heart disease (CHD) (Am J Trans Res (2019) 11(6):3610-3619).

miR-143-3p regulated both the cellular response of pulmonary vascular cells and the exosome-mediated response; however, inhibition of miR-143-3p blocked experimental pulmonary hypertension (Circ Res. (2015) 117:870-883).

miR-145 and miR-143 are direct transcriptional targets of serum response factor, myocardin, and Nkx2-5 (NK2 transcription factor-related, locus 5), and are downregulated in damaged or atherosclerotic vessels that include proliferating, less-differentiated smooth muscle cells. miR-145 and miR-143 cooperatively target a network of transcription factors such as Klf4 (Kruppel-like factor 4), myocardin, and Elk-1 (ELK1, a member of the ETS oncogene family), promote smooth muscle differentiation, and suppress proliferation (Nature (2009) 460, 705-710).

### (2-3) Concerning hsa-miR-221-3p

miR-221 exhibits notable myocardial protective and anti-apoptotic characteristics, and is believed to be beneficial for enhancing the efficiency of cardiomyocyte transplantation and cardiac regeneration (BioMed Research International (2015) volume 2015, 354517, 18 pages).

The fact that human blood-derived progenitor cells, endothelial progenitor cells, umbilical vein endothelial cells (ECs), and quiescent endothelial cells express miR-221/222 at high levels, suggests an important role of this miRNA cluster in endothelial (patho)physiology (Curr. Vasc. Pharmacol. (2017) 15(1):40-46).

### (2-4) In relation to hsa-miR-30a-3p and hsa-miR-30a-5p

Apo E-/- mice treated with miR-30a-5p-ago exhibited significantly reduced aortic and aortic root lesion areas, decreased lipoprotein and inflammatory cytokine levels, and increased anti-inflammatory cytokine levels. In Apo E-/- mice treated with miR-30a-5p-ago and LPS-treated RAW 264.7 macrophages, the ratio of M1/M2 macrophages was reduced by controlling Smad-1/2 phosphorylation (Mol. Ther. Nucleic Acids (2021) 26:1303-1317).

HPostC protected the survival of senescent cardiomyocytes against H/R injury through DNMT3b-dependent activation of miR-30a. miR-30a may be a therapeutic target for ischemic myocardial infarction (Circ. J. (2020) 84:616-625).

In the present invention, when the small extracellular vesicles contain a miRNA targeting a gene involved in the expression of thrombospondin 1 (preferably, TSP1 gene) as a target gene, the small extracellular vesicles preferably function as an expression controlling agent for a gene involved in the expression of thrombospondin 1. It is preferable that the small extracellular vesicles are a thrombospondin 1 expression inhibitor.

In this case, it is preferable that the small extracellular vesicles contain at least one type of hsa-let-7a-5p, hsa-let-7b-5p, hsa-let-7f-5p, hsa-miR-132-3p, hsa-miR-143-3p, hsa-miR-19a-3p, hsa-miR-19b-3p, hsa-miR-221-3p, hsa-miR-30a-3p, and hsa-miR-30a-5p; it is more preferable that the small extracellular vesicles contain at least hsa-miR-19a-3p, hsa-miR-19b-3p, and hsa-miR-221-3p; and it is particularly preferable that the small extracellular vesicles contain all of hsa-let-7a-5p, hsa-let-7b-5p, hsa-let-7f-5p, hsa-miR-132-3p, hsa-miR-143-3p, hsa-miR-19a-3p, hsa-miR-19b-3p, hsa-miR-221-3p, hsa-miR-30a-3p, and hsa-miR-30a-5p.

It is preferable that the small extracellular vesicles contain at least one type of miRNAs targeting genes involved in the expression of thrombospondin 1 as target genes such that the Log2Ratio of the number of lead counts obtained by analysis using IMOTA is 2.0 or more, more preferably 4.0 or more, and particularly preferably 6.0 or more. It is preferable that the small extracellular vesicles contain hsa-miR-19a-3p, hsa-miR-19b-3p, and hsa-miR-221-3p such that the Log2Ratios of the number of lead counts obtained by analysis using IMOTA are each independently 2.0 or more, more preferably 4.0 or more, and particularly preferably 5.0 or more. It is more preferable that the small extracellular vesicles contain hsa-miR-221-3p such that the Log2Ratio is 12.0 or more.

With regard to the small extracellular vesicles, it is preferable that the expression level of at least one type of hsa-miR-19a-3p, hsa-miR-19b-3p, and hsa-miR-221-3p is 1.1 times or more, more preferably 1.5 times or more, and particularly preferably 2 times or more, as compared with exosomes obtained from a culture supernatant of adipose-derived stem cells or exosomes obtained from a culture supernatant of umbilical cord-derived stem cells.

When the small extracellular vesicles are a thrombospondin 1 expression inhibitor, it is preferable that the expression of the gene of thrombospondin 1 in any cell can be suppressed to be 0.8-fold or less, more preferably 0.7-fold or less, and particularly preferably 0.6-fold or less, the normal level (the case of untreated cells).

### (Type of miRNA)

Here, the small extracellular vesicles derived from a culture supernatant of dental pulp-derived stem cells contain about 2600 types of small RNAs. Among these, about 1800 types are miRNAs. Among these miRNAs, 180 to 200 types of miRNAs are present in high amounts. The miRNAs present in high amounts in the small extracellular vesicles derived from dental pulp-derived stem cells have a feature that they include many microRNAs involved in the treatment of cranial nerve diseases and vasculitis, and this feature is a finding that was conventionally not known and has been newly discovered by the present inventor. This feature is greatly different from the types of miRNAs that are present in high amounts in the small extracellular vesicles of other mesenchymal stem cells. For example, miRNAs that are present in high amounts in the small extracellular vesicles of adipose-derived stem cells and the small extracellular vesicles of umbilical cord-derived stem cells hardly include microRNAs that are involved in the treatment of cranial nerve diseases and vasculitis.

It is preferable that the small extracellular vesicles contain two or more types, more preferably 10 types or more, even more preferably 30 types or more, particularly preferably 50 types or more, and more particularly preferably 100 types or more, of microRNAs capable of controlling the expression of proteins and/or genes involved in vasculitis (hereinafter, also referred to as vasculitis-related microRNAs).

### (Type of small extracellular vesicles)

It is preferable that the small extracellular vesicles are at least one type selected from the group consisting of exosomes, microvesicles, membrane particles, membrane vesicles, ectosomes, and exovesicles or microvesicles, and it is more preferable that the small extracellular vesicles are exosomes.

The diameter of the small extracellular vesicles is preferably 10 to 1000 nm, more preferably 30 to 500 nm, and particularly preferably 50 to 150 nm.

Furthermore, it is desirable that molecules called tetraspanins, such as CD9, CD63, and CD81, are present on the surface of the small extracellular vesicles, and those may be CD9 alone, CD63 alone, or CD81 alone, or may be any combination of two or three of those.

Preferred embodiments in the case of using exosomes as the small extracellular vesicles will be described below; however, the small extracellular vesicles used in the present invention are not limited to exosomes.

It is preferable that exosomes are extracellular vesicles released from cells at the time of fusion of multivesicular bodies with the protoplasmic membrane.

It is preferable that the surface of the exosomes include lipids and proteins derived from the cell membranes of dental pulp-derived stem cells.

It is preferable that the inside of the exosomes contain intracellular substances of dental pulp-derived stem cells, such as nucleic acids (microRNA, messenger RNA, DNA, and the like) and proteins.

Exosomes are known to be used for cell-to-cell communication by transportation of genetic information from a certain cell to another cell. Exosomes are easily traceable and can be targeted to specific areas.

### (Content of small extracellular vesicles)

The content of the small extracellular vesicles in the small extracellular vesicle composition is not particularly limited. It is preferable that the small extracellular vesicle composition contains 0.5×10⁸ or more small extracellular vesicles, more preferably 1.0×10⁸ or more small extracellular vesicles, particularly preferably 2.0×10⁸ or more small extracellular vesicles, more particularly preferably 2.5×10⁸ or more small extracellular vesicles, and even more particularly preferably 1.0×10⁹ or more small extracellular vesicles.

Furthermore, the content concentration of the small extracellular vesicles in the small extracellular vesicle composition is not particularly limited. It is preferable that the small extracellular vesicle composition includes 1.0×10⁸ small extracellular vesicles/mL or more, more preferably 2.0×10⁸ small extracellular vesicles/mL or more, particularly preferably 4.0×10⁸ small extracellular vesicles/mL or more, more particularly preferably 5.0×10⁸ small extracellular vesicles/mL or more, and even more particularly preferably 2.0×10⁹ small extracellular vesicles/mL or more.

A preferred embodiment of the expression controlling agent of the present invention can maintain a high level of a miRNA targeting a gene involved in the expression of PTX3 as a target gene, or a miRNA targeting a gene involved in the expression of TSP-1 as a target gene, by including such a large amount or high concentration of the small extracellular vesicles.

### <Other components>

The small extracellular vesicle composition may contain other components, in addition to the small extracellular vesicles, depending on the type or purpose of the animal as a target of administration, to the extent that does not impair the effects of the present invention. Examples of the other components include a nutritional component, an antibiotic substance, a cytokine, a protective agent, a carrier, an excipient, a disintegrant, a buffer agent, an emulsifier, a suspending agent, a soothing agent, a stabilizer, a preservative, and an antiseptic agent.

Examples of the nutritional component include fatty acids and vitamins.

Examples of the antibiotic substance include penicillin, streptomycin, and gentamycin.

Examples of the carrier include materials known as pharmaceutically acceptable carriers.

The small extracellular vesicle composition may be a culture supernatant itself of dental pulp-derived stem cells or small extracellular vesicles themselves, or may be a pharmaceutical composition further containing a pharmaceutically acceptable carrier, excipient, and the like. The purpose of the pharmaceutical composition is to promote the administration of small extracellular vesicles to a target of administration.

The pharmaceutically acceptable carrier is preferably a carrier (including a diluent) that does not cause significant irritation in the target of administration, and does not suppress the biological activity and characteristics of the compound to be administered. Examples of the carrier include propylene glycol; (physiological) saline; an emulsion; a buffer solution; a medium, for example, DMEM or RPMI; and a cryopreservation medium containing components that scavenge free radicals.

The small extracellular vesicle composition may include an active ingredient of a conventionally known therapeutic drug for vasculitis or skin hardening associated with rheumatoid arthritis and autoimmune diseases. Those skilled in the art can make appropriate modifications depending on the use application, the target of administration, and the like.

On the other hand, it is preferable that the small extracellular vesicle composition does not include predetermined substances.

For example, it is preferable that the small extracellular vesicle composition does not include dental pulp-derived stem cells.

Furthermore, it is preferable that the small extracellular vesicle composition does not include MCP-1. However, the small extracellular vesicle composition may include a cytokine other than MCP-1. Examples of the other cytokine include those described in [0014] to [0020] of JP2018-023343A.

Furthermore, it is preferable that the small extracellular vesicle composition does not include Siglec-9. However, the small extracellular vesicle composition may include another sialic acid-binding immunoglobulin-like lectin other than Siglec-9.

Incidentally, it is preferable that the small extracellular vesicle composition substantially does not include serum (fetal bovine serum, human serum, sheep serum, or the like). Furthermore, it is preferable that the small extracellular vesicle composition substantially does not include a conventional serum substitute such as Knockout serum replacement (KSR).

In the small extracellular vesicle composition, the contents (solid contents) of the above-described other components are all preferably 1% by mass or less, more preferably 0.1% by mass or less, and particularly preferably 0.01% by mass or less.

### <Method for producing small extracellular vesicles>

The method for producing small extracellular vesicles is not particularly limited.

The PTX3 expression controlling agent of the present invention may be prepared by preparing a culture supernatant of dental pulp-derived stem cells or the like, and subsequently purifying small extracellular vesicles from the culture supernatant of the dental pulp-derived stem cells. Alternatively, the PTX3 expression controlling agent of the present invention may be prepared by purifying small extracellular vesicles from a commercially available culture supernatant of dental pulp-derived stem cells. In addition, the PTX3 expression controlling agent of the present invention may also be prepared by receiving a composition including a culture supernatant of dental pulp-derived stem cells that has been discarded (or appropriately purifying the composition), and purifying small extracellular vesicles therefrom.

### (Method for preparing culture supernatant of dental pulp-derived stem cells or the like)

The culture supernatant of dental pulp-derived stem cells or the like is not particularly limited.

It is preferable that the culture supernatant of dental pulp-derived stem cells or the like substantially does not include serum. For example, in the culture supernatant of dental pulp-derived stem cells or the like, the content of the serum is preferably 1% by mass or less, more preferably 0.1% by mass or less, and particularly preferably 0.01% by mass or less.

The dental pulp-derived stem cells may be cells derived from a human or an animal other than a human. Examples of the animal other than a human include the same animals (biological species) as the target to which the PTX3 expression controlling agent of the present invention is administered, that will be described below, and a mammal is preferred.

The dental pulp-derived stem cells used for the culture supernatant are not particularly limited. Dental pulp stem cells from exfoliated deciduous teeth (stem cells from exfoliated deciduous teeth), dental pulp stem cells from deciduous teeth obtained by other methods, or dental pulp stem cells from permanent teeth (DPSCs) can be used. In addition to dental pulp stem cells from human deciduous teeth and dental pulp stem cells from human permanent teeth, dental pulp-derived stem cells derived from an animal other than human, such as dental pulp stem cells from porcine deciduous teeth, can be used.

In addition to exosomes, dental pulp-derived stem cells can produce a variety of cytokines such as vascular endothelial growth factor (VEGF), hepatocyte growth factor (HGF), insulin-like growth factor (IGF), platelet-derived growth factor (PDGF), transforming growth factor-beta (TGF-β)-1 and (TGF-β)-3, TGF-α, KGF, HBEGF, SPARC, other growth factors, and chemokines. Furthermore, the dental pulp-derived stem cells can produce many other physiologically active substances.

In the present invention, it is particularly preferable that the dental pulp-derived stem cells used in a culture supernatant of dental pulp-derived stem cells are dental pulp-derived stem cells containing many proteins, and it is preferable to use dental pulp stem cells from deciduous teeth. That is, in the present invention, it is preferable to use a culture supernatant of dental pulp stem cells from deciduous teeth.

The dental pulp-derived stem cells used in the present invention may be naturally occurring stem cells or may be genetically modified stem cells, as long as they can achieve the intended treatment.

Particularly in the present invention, immortalized stem cells of dental pulp-derived stem cells can be used. By using immortalized stem cells that are capable of substantially unlimited proliferation, the amount and composition of biological factors included in the culture supernatant of stem cells can be stabilized over a long period of time. The immortalized stem cells of dental pulp-derived stem cells are not particularly limited. However, it is preferable that the immortalized stem cells are immortalized stem cells that are not cancerized. The immortalized stem cells of dental pulp-derived stem cells can be prepared by adding the following low-molecular weight compounds (inhibitors) alone or in combination to dental pulp-derived stem cells and culturing the cells.

A TGF β receptor inhibitor is not particularly limited as long as it has an action of inhibiting the function of the transforming growth factor (TGF) β receptor, and examples thereof include 2-(5-benzo[1,3]dioxol-4-yl-2-tert-butyl-1H-imidazol-4-yl)-6-methylpyridine, 3-(6-methylpyridin-2-yl)-4-(4-quinolyl)-1-phenylthiocarbamoyl-1H-pyrazole (A-83-01), 2-[(5-chloro-2-fluorophenyl)pteridin-4-yl]pyridin-4-ylamine (SD-208), 3-[(pyridin-2-yl)-4-(4-quinonyl)]-1H-pyrazole, 2-(3-(6-methylpyridin-2-yl)-1H-pyrazol-4-yl)-1,5-naphthyridine (all from Merck & Co., Inc.), and SB431542 (Sigma-Aldrich Corporation). A preferred TGF β receptor inhibitor may be A-83-01.

A ROCK inhibitor is not particularly limited as long as it has an action of inhibiting the function of Rho-binding kinase. Examples of the ROCK inhibitor include GSK269962A (Axon Medchem BV), Fasudil hydrochloride (Tocris Bioscience, Ltd.), Y-27632, and H-1152 (both from FUJIFILM Wako Pure Chemical Corporation). A preferred ROCK inhibitor may be Y-27632.

A GSK3 inhibitor is not particularly limited as long as it inhibits GSK-3 (Glycogen synthase kinase 3), and examples thereof include A1070722, BIO, and BIO-acetoxime (all from TOCRIS Cookson, Ltd.).

An MEK inhibitor is not particularly limited as long as it has an action of inhibiting the function of MEK (MAP kinase-ERK kinase), and examples thereof include AZD6244, CI-1040 (PD184352), PD0325901, RDEA119 (BAY86-9766), SL327, U0126-EtOH (all from Selleck Chemicals LLC), PD98059, U0124, and U0125 (all from Cosmo Bio Co., Ltd.).

In a case where the PTX3 expression controlling agent of the present invention is used for regenerative medicine, due to the requirements of the Act on Safety Assurance of Regenerative Medicine, a culture supernatant of dental pulp-derived stem cells or immortalized stem cells thereof, and a composition including small extracellular vesicles derived from the culture supernatant are in a state that does not contain any other somatic stem cells other than dental pulp-derived stem cells or the like. The small extracellular vesicle composition may contain mesenchymal stem cells or other somatic stem cells other than dental pulp-derived stem cells or the like; however, it is preferable that the small extracellular vesicle composition does not contain those cells.

Examples of the other somatic stem cells other than mesenchymal stem cells include stem cells derived from the dermal system, digestive system, bone marrow system, and nervous system, but are not limited to these. Examples of somatic stem cells in the dermal system include epithelial stem cells and hair follicle stem cells. Examples of somatic stem cells in the digestive system include pancreatic (general) stem cells and hepatic stem cells. Examples of somatic stem cells in the bone marrow system (other than mesenchymal stem cells) include hematopoietic stem cells. Examples of somatic stem cells in the nervous system include neural stem cells and retinal stem cells.

The small extracellular vesicle composition may contain stem cells other than somatic stem cells; however, it is preferable that the small extracellular vesicle composition does not contain those cells. Examples of the stem cells other than somatic stem cells include embryonic stem cells (ES cells), induced pluripotent stem cells (iPS cells), and embryonic carcinoma cells (EC cells).

The method for preparing a culture supernatant of dental pulp-derived stem cells or immortalized stem cells thereof is not particularly limited, and any conventional method can be used.

The culture supernatant of dental pulp-derived stem cells or the like is a culture fluid obtained by culturing dental pulp-derived stem cells. A culture supernatant that can be used in the present invention can be obtained by, for example, separating and removing cell components after culturing of dental pulp-derived stem cells. Culture supernatants that have been appropriately subjected to various treatments (for example, a centrifugation, concentration, solvent replacement, dialysis, freezing, drying, freeze-drying, dilution, desalting, and preservation) may also be used.

The dental pulp-derived stem cells for obtaining a culture supernatant of dental pulp-derived stem cells can be selected by a conventional method, and can be selected on the basis of the size or morphology of the cells, or as adhesive cells. The dental pulp-derived stem cells can be selected from dental pulp cells collected from exfoliated deciduous teeth or permanent teeth, as adhesive cells or their passage cells. As the culture supernatant of dental pulp-derived stem cells, a culture supernatant obtained by culturing the selected stem cells can be used.

Incidentally, the "culture supernatant of dental pulp-derived stem cells or the like" is preferably a culture fluid that does not include the cells themselves obtained by culturing dental pulp-derived stem cells or the like. According to an embodiment, it is preferable that the culture supernatant of dental pulp-derived stem cells used in the present invention does not include any cells (regardless of the type of cells) as a whole. The composition of this embodiment is clearly distinguished definitely from dental pulp-derived stem cells themselves, as well as from various compositions including dental pulp-derived stem cells, due to this feature. A typical example of this embodiment is a composition that does not include dental pulp-derived stem cells and is composed only of a culture supernatant of dental pulp-derived stem cells.

The culture supernatant of dental pulp-derived stem cells used in the present invention may include culture supernatants of both dental pulp-derived stem cells from deciduous teeth and adult dental pulp-derived stem cells. It is preferable that the culture supernatant of dental pulp-derived stem cells used in the present invention includes a culture supernatant of dental pulp-derived stem cells from deciduous teeth as an active ingredient, and it is more preferable that the culture supernatant includes 50% by mass or more, and even more preferably 90% by mass or more, of the culture supernatant of dental pulp-derived stem cells from deciduous teeth. It is more particularly preferable that the culture supernatant of dental pulp-derived stem cells used in the present invention is a composition composed only of a culture supernatant of dental pulp-derived stem cells from deciduous teeth.

As the culture fluid of dental pulp-derived stem cells for obtaining a culture supernatant, a basal medium, a medium to which serum and the like have been added to a basal medium, or the like can be used. Incidentally, as the basal medium, in addition to Dulbecco's modified Eagle's medium (DMEM), Iscove's modified Dulbecco's medium (IMDM) (GIBCO, Inc. and the like), Ham's F12 medium (HamF12) (SIGMA-ALDRICH Corporation, GIBCO, Inc., and the like), RPMI1640 medium, and the like can be used. Furthermore, examples of the components that can be added to the medium include sera (fetal bovine serum, human serum, sheep serum, and the like), serum substitutes (Knockout serum replacement (KSR) and the like), bovine serum albumin (BSA), antibiotic substances, various vitamins, and various minerals.

However, in order to prepare the "culture supernatant of dental pulp-derived stem cells" that does not include serum, a serum-free medium may be used through the entire process or for the final subculture or several subcultures previous to the final one. For example, a culture supernatant of dental pulp-derived stem cells that do not include serum can be prepared by culturing dental pulp-derived stem cells in a medium that does not include serum (serum-free medium). By conducting a single subculture or several subcultures, and performing the final subculture or several subcultures previous to the final one in a serum-free medium, a culture supernatant of dental pulp-derived stem cells or the like, which does not include serum, can be obtained. On the other hand, a culture supernatant of dental pulp-derived stem cells that does not include serum can be obtained by removing serum from a collected culture supernatant by utilizing dialysis, solvent replacement using a column, or the like.

For the culturing of dental pulp-derived stem cells for obtaining a culture supernatant, conditions that are commonly used can be applied as they are. The method for preparing a culture supernatant of dental pulp-derived stem cells may be carried out in the same manner as in the cell culturing method that will be described below, except that the steps of isolating and selecting stem cells are appropriately adjusted depending on the type of the stem cells. Those skilled in the art can appropriately carry out isolation and selection of dental pulp-derived stem cells in accordance with the type of the dental pulp-derived stem cells.

Furthermore, for the culturing of dental pulp-derived stem cells, special conditions may be applied in order to produce a large quantity of small extracellular vesicles such as exosomes. Examples of the special conditions include low temperature conditions, low oxygen conditions, microgravity conditions, and conditions of co-culturing with some kind of stimuli.

The culture supernatant of dental pulp-derived stem cells used for preparing small extracellular vesicles such as exosomes in the present invention may include other components in addition to the culture supernatant of dental pulp-derived stem cells; however, it is preferable that the culture supernatant substantially does not include other components.

However, various types of additives used for preparing exosomes may be added to the culture supernatant of dental pulp-derived stem cells, and then the culture supernatant may be stored.

### (Preparation of small extracellular vesicles)

Small extracellular vesicles can be prepared by purifying small extracellular vesicles from the culture supernatant of dental pulp-derived stem cells or the like.

It is preferable that the purification of small extracellular vesicles is separation of a fraction including the small extracellular vesicles from the culture supernatant of dental pulp-derived stem cells, and it is more preferable that the purification is isolation of the small extracellular vesicles.

The small extracellular vesicles may be isolated by separating the small extracellular vesicles from non-associated components based on the characteristics of the small extracellular vesicles. For example, the small extracellular vesicles may be isolated on the basis of molecular weight, size, morphology, composition, or biological activity.

In the present invention, the small extracellular vesicles can be purified by preparatively separating a specific fraction (for example, a precipitate) rich in the small extracellular vesicles, which has been obtained by centrifuging the culture supernatant of dental pulp-derived stem cells. Unnecessary components (insoluble components) in fractions other than a predetermined fraction may be removed. The removal of the solvent and dispersion medium, and any unnecessary components from the small extracellular vesicle composition does not have to be complete removal. An example of the conditions for centrifugation is 100 to 20000 g for 1 to 30 minutes.

In the present invention, the small extracellular vesicles can be purified by subjecting the culture supernatant of dental pulp-derived stem cells or a centrifugation product thereof to a filtration treatment. Unnecessary components can be removed by a filtration treatment. Furthermore, when a filtration membrane having an appropriate pore size is used, the removal of unnecessary components and a sterilization treatment can be carried out simultaneously. The material, pore size, and the like of the filtration membrane used for the filtration treatment are not particularly limited. Filtration can be carried out using a known method with a filtration membrane having an appropriate molecular weight or size cutoff. From the viewpoint of easily preparatively separating exosomes, the pore size of the filtration membrane is preferably 10 to 1000 nm, more preferably 30 to 500 nm, and particularly preferably 50 to 150 nm.

In the present invention, the culture supernatant of dental pulp-derived stem cells or a centrifugation product thereof or a filtration product of the culture supernatant or the centrifugation product can be separated using further separation means such as column chromatography. For example, high performance liquid chromatography (HPLC) using various columns can be used. Regarding the column, a size exclusion column or a binding column can be used.

In order to track the small extracellular vesicles (or activity thereof) in each fraction at each treatment stage, one or more characteristics or biological activities of the small extracellular vesicles can be used. For example, light scattering, refractive index, dynamic light scattering, or UV-visible detectors can be used to track small extracellular vesicles. Alternatively, specific enzyme activity and the like can be used in order to track the activity in each fraction.

As the method for purifying small extracellular vesicles, the method described in [0034] to [0064] of JP2019-524824A may be used, the content of which is incorporated herein by reference.

The final form of the small extracellular vesicle composition is not particularly limited. For example, the small extracellular vesicle composition may be in a form in which the small extracellular vesicles are packed in a container together with a solvent or a dispersion medium; a form in which the small extracellular vesicles are gelled together with a gel and packed in a container; or a form in which the small extracellular vesicles are frozen and/or dried to solidify, and then formulated or packed in a container. Examples of the container include a tube, a centrifuge tube, and a bag suitable for cryopreservation. The freezing temperature can be set to, for example, -20°C to -196°C.

The PTX3 expression controlling agent of the present invention has advantages such as ease of mass production, utilization of culture fluids of stem cells that have been conventionally discarded as industrial waste or the like, and reduction of disposal cost of culture fluids of stem cells, as compared with compositions that can be used as conventional therapeutic drugs or prophylactic drugs for vasculitis or skin hardening associated with rheumatoid arthritis and autoimmune diseases. In particular, in a case where the culture supernatant of dental pulp-derived stem cells is a culture supernatant of human dental pulp-derived stem cells, there is also an advantage that when the PTX3 expression controlling agent of the present invention is applied to humans, the culture supernatant is highly safe from an immunological viewpoint, and there are fewer ethical problems. In a case where the culture supernatant of dental pulp-derived stem cells is a culture supernatant of dental pulp-derived stem cells from a patient with vasculitis, it will be safer when the PTX3 expression controlling agent of the present invention is applied to the patient, and there will be fewer ethical problems.

In a case where the PTX3 expression controlling agent of the present invention is derived from a culture supernatant of dental pulp-derived stem cells, the PTX3 expression controlling agent is also used for applications in restorative medicine. In particular, a composition including small extracellular vesicles derived from a culture supernatant of dental pulp-derived stem cells or the like is preferably used for applications in restorative medicine. Here, in regenerative medicine assumed to be based on stem cell transplantation, it is known that stem cells do not play the main role in regeneration, and liquid components produced by stem cells repair organs together with the patients' own stem cells. Difficult problems associated with conventional stem cell transplantation, such as cancerization, standardization, method of administration, storage property, and culturing method, are solved, and restorative medicine is made possible by a culture supernatant of dental pulp-derived stem cells or a composition using small extracellular vesicles derived from the supernatant. As compared with stem cell transplantation, when the PTX3 expression controlling agent of the present invention is used, tumor formation or the like is less likely to occur because cells are not transplanted, and it can be said to be safer. Furthermore, the PTX3 expression controlling agent of the present invention has an advantage that agents with uniformly standardized quality can be used. Since mass production and efficient method of administration can be selected, the PTX3 expression controlling agent can be utilized at low cost.

### [Prophylactic drug or therapeutic drug for vasculitis or skin hardening associated with rheumatoid arthritis and autoimmune diseases]

A prophylactic drug or therapeutic drug for vasculitis or skin hardening associated with rheumatoid arthritis and autoimmune diseases of the present invention includes the PTX3 expression controlling agent of the present invention.

The term "prophylactic" as used in the present specification refers to preventing the onset of a disease (vasculitis in the present specification) before the disease occurs. Furthermore, the term "therapeutic" as used in the present specification refers to alleviating, suppressing, or inhibiting the progression of symptoms of a developed disease, and improving the symptoms.

### [Method for improving vasculitis or skin hardening associated with rheumatoid arthritis and autoimmune diseases]

A method for improving vasculitis or skin hardening associated with rheumatoid arthritis and autoimmune diseases of the present invention includes administering an effective amount of the PTX3 expression controlling agent of the present invention or an effective amount of the prophylactic drug or therapeutic drug for vasculitis or skin hardening associated with rheumatoid arthritis and autoimmune diseases of the present invention, to a subject who has developed vasculitis.

The step of administering the PTX3 expression controlling agent and the like of the present invention to a subject who has developed vasculitis or skin hardening associated with rheumatoid arthritis and autoimmune diseases is not particularly limited.

Examples of the method for administration include spraying or inhalation into the oral cavity, nasal cavity, or airways, infusion, local administration, nasal drops, and application on the skin, and a minimally invasive method is preferred. The method for local administration is preferably injection. Furthermore, electroporation in which a voltage (electrical pulse) is applied to the skin surface to temporarily create fine pores in the cell membrane, and an active ingredient is allowed to penetrate into the dermis layer, which cannot be reached in normal care, is also preferable. **In** the case of local administration, examples of administration include intravenous administration, intraarterial administration, intraportal administration, intradermal administration, subcutaneous administration, intramuscular administration, and intraperitoneal administration, and the local administration is more preferably intraarterial administration, intravenous administration, subcutaneous administration, or intraperitoneal administration. In a case where the PTX3 expression controlling agent of the present invention is administered to a subject who has developed skin hardening, it is more preferable that the administration is conducted by application on the skin. For example, it is preferable to blend the PTX3 expression controlling agent of the present invention into skin application agents such as a skin toner (lotion), a beauty essence, a milky lotion, a cream, an ointment, and a pack, and apply these on the skin. When the prophylactic drug or therapeutic drug for vasculitis or skin hardening associated with rheumatoid arthritis and autoimmune diseases of the present invention is a prophylactic drug or therapeutic drug for skin hardening, the prophylactic drug or therapeutic drug is preferably a skin application agent.

Furthermore, the in vivo distribution of the small extracellular vesicles can be altered using various formulation techniques. A number of methods for altering the in vivo distribution are known to those skilled in the art. Examples of such a method include protection of exosomes in vesicles composed of substances such as proteins, lipids (for example, liposomes), carbohydrates, or synthetic polymers.

The PTX3 expression controlling agent of the present invention administered to a subject who has developed vasculitis or skin hardening associated with rheumatoid arthritis and autoimmune diseases may circulate within the body of the subject and reach a predetermined tissue. There are no particular limitations on the frequency of administration and the interval of administration. The frequency of administration can be set to one or more times per week, and the frequency of administration is preferably 5 or more times, more preferably 6 or more times, and particularly preferably 7 or more times. The interval of administration is preferably one hour to one week, more preferably half a day to one week, and particularly preferably one day (once a day). However, the interval of administration can be appropriately adjusted depending on the biological species of the target of administration and the symptoms of the target of administration.

It is preferable that the PTX3 expression controlling agent of the present invention is used for administering the small extracellular vesicles to a subject who has developed vasculitis, one or more times a week over the effective therapeutic period. When the target of administration is a human, it is more preferable that the frequency of administration per week is higher, and it is preferable to administer five or more times a week over the effective therapeutic period, while daily administration is preferred.

In the case of using a culture supernatant of dental pulp-derived stem cells at a concentration of 2.0×10⁹ small extracellular vesicles/ml, in a mouse model, the dose is preferably 0.1 to 5 ml, more preferably 0.3 to 3 ml, and particularly preferably 0.5 to 1 ml, per mouse (about 25 g).

In the case of using small extracellular vesicles at a concentration of 0.1×10⁸ small extracellular vesicles/µg, in a mouse model, the dose is preferably 1 to 50 µg, more preferably 3 to 30 µg, and more particularly preferably 5 to 25 µg, per mouse (about 25 g).

Preferable ranges of the amount of administration per body weight for other animals can be calculated using the proportional relationship from the amount of administration per body weight of a model mouse (about 25 g). However, the amount of administration can be appropriately adjusted depending on the symptoms of the target of administration.

The target animal (biological species) to which the PTX3 expression controlling agent of the present invention is administered is not particularly limited. The target animal to which the PTX3 expression controlling agent of the present invention is administered is preferably a mammal, a bird (chicken, quail, duck, or the like), or fish (salmon, trout, tuna, bonito, or the like). The mammal may be a human or a non-human mammal; however, it is particularly preferable that the mammal is a human. The non-human mammal is more preferably a cow, a pig, a horse, a goat, a sheep, a monkey, a dog, a cat, a mouse, a rat, a guinea pig, or a hamster.

The PTX3 expression controlling agent of the present invention may be used in combination with a conventionally known therapeutic drug for vasculitis or skin hardening associated with rheumatoid arthritis and autoimmune diseases. Specifically, for example, the PTX3 expression controlling agent may be used in combination with conventionally known steroids, immunosuppressants, biological preparations (tocilizumab, TNF inhibitor, and the like), antiplatelet drugs, antihistamine drugs, antiallergic drugs, NSAIDs (non-steroid anti-inflammatory drugs).

### EXAMPLES

The features of the present invention will be described more specifically below by way of Examples, Comparative Examples, or Reference Examples. The materials, amounts used, proportions, contents of treatments, treatment procedures, and the like shown in the following Examples can be appropriately modified as long as the gist of the present invention is maintained. Therefore, the scope of the present invention should not be construed as being limited by the following specific examples.

### [Example 1]

### <Preparation of culture supernatant of dental pulp-derived stem cells>

A culture supernatant of dental pulp stem cells from human deciduous teeth was prepared according to the method described in Example 6 of Japanese Patent No. 6296622, except that DMEM medium was used instead of the DMEM/HamF 12 mixed medium, and the culture supernatant was preparatively separated. In the primary culture, the cells were cultured after fetal bovine serum (FBS) was added, whereas in the subcultures, a supernatant of subculture fluids obtained by culturing the cells using a primary culture fluid was preparatively separated such that the supernatant did not include FBS, and the culture supernatant of dental pulp stem cells from deciduous teeth was prepared. DMEM is Dulbecco's modified Eagle's medium, and F12 is Ham's F12 medium.

### <Preparation of exosomes>

Exosomes of dental pulp-derived stem cells were purified from the obtained culture supernatant of dental pulp-derived stem cells, by the following method.

The culture supernatant (100 mL) of dental pulp stem cells from deciduous teeth was filtered through a filter having a pore size of 0.22 micrometers, and then the solution was centrifuged for 60 minutes at 4°C at 100000×g. The supernatant was decanted, and exosome-enriched pellets were resuspended in phosphate-buffered saline (PBS). A resuspended sample was centrifuged at 100000×g for 60 minutes. Pellets were collected again from the bottom of the centrifuge tube as a concentrated sample (approximately 100 µl). The protein concentration was determined using a micro BCA protein assay kit (Pierce, Rockford, IL). A composition (concentrated solution) including exosomes was stored at -80°C.

The composition including exosomes purified from the culture supernatant of dental pulp-derived stem cells was obtained as an expression controlling agent (small extracellular vesicle composition sample) of Example 1.

The average particle size and concentration of the small extracellular vesicles included in the expression controlling agent of Example 1 were evaluated using a nanoparticle analysis system NanoSight (manufactured by Quantum Design Japan, Inc.).

The average particle size of the small extracellular vesicles included in the expression controlling agent of Example 1 was 50 to 150 nm.

The expression controlling agent of Example 1 was a high-concentration exosome solution having a concentration of 1.0×10⁹ exosomes/ml or more, and specifically, the expression controlling agent was a high-concentration exosome solution having a concentration of 2.0×10⁹ exosomes/ml.

Furthermore, the components of the obtained expression controlling agent of Example 1 were analyzed by a known method. As a result, it was found that the expression controlling agent of Example 1 does not contain stem cells of dental pulp-derived stem cells, does not contain MCP-1, and does not contain Siglec-9. Therefore, it was found that an active ingredient different from MCP-1 and Siglec-9, which are active ingredients of the culture supernatant of mesenchymal stem cells, and analogues thereof, is the active ingredient of the expression controlling agent of Example 1.

### [Comparative Example 1]

### <Preparation of culture supernatant of umbilical cord-derived stem cells>

A culture supernatant of umbilical cord-derived stem cells was prepared in the same manner as in Example 1, except that human umbilical cord-derived stem cells were used instead of dental pulp stem cells from deciduous teeth, and a composition containing exosomes purified from the umbilical cord-derived stem cells (small extracellular vesicle composition of Comparative Example 1) was prepared.

### [Test Example 1]: MicroRNA expressed in exosomes

The small RNA contained in the small extracellular vesicle composition of Example 1 was analyzed by next-generation sequencing (NGS) analysis. By the NGS analysis, 1787 miRNAs contained in the small extracellular vesicle composition of Example 1 (exosomes of dental pulp-derived stem cells) were identified. The obtained results are shown in the following Table 1.

**[Table 1]**

| name | Number of detections | % |
|---|---|---|
| miRNA | 1787 | 72.35 |
| piRNA | 123 | 4.98 |
| tRNA | 411 | 16.64 |
| other RNA | 149 | 6.03 |
| Total number | 2470 | 100 |

### [Test Example 2]: Search for disease-related microRNAs

A search for disease-related microRNAs was conducted. Extraction of disease-related miRNAs was carried out using IMOTA (Interactive Multi-Omics-Tissue Atlas). IMOTA is an interactive multi-omics atlas that allows investigation on the interactions and expression levels of miRNA, mRNA, and proteins in each tissue and cell (Nucleic Acids Research, Volume 46, Issue D1, 4 January 2018, Pages D770-D775, "IMOTA: an interactive multi-omics tissue atlas for the analysis of human miRNA-target interactions"). Here, a search was conducted to see whether microRNAs that controlled proteins and/or genes involved in vasculitis or skin hardening associated with rheumatoid arthritis and autoimmune diseases were included, or whether microRNAs that controlled proteins and/or genes that were targets of therapeutic drugs were included. In this Test Example 2, a search was conducted for miRNAs targeting genes involved in the expression of PTX3 as target genes. Additionally, for reference, a search was also conducted for miRNAs targeting genes involved in the expression of TSP-1 as target genes.

### [Test Example 2]: Search for disease-related microRNAs

A search for disease-related microRNAs was conducted. Extraction of disease-related miRNAs was carried out using IMOTA (Interactive Multi-Omics-Tissue Atlas). IMOTA is an interactive multi-omics atlas that allows investigation on the interactions and expression levels of miRNA, mRNA, and proteins in each tissue and cell (Nucleic Acids Research, Volume 46, Issue D1, 4 January 2018, Pages D770-D775, "IMOTA: an interactive multi-omics tissue atlas for the analysis of human miRNA-target interactions"). Here, a search was conducted to see whether microRNAs that controlled proteins and/or genes involved in vasculitis or skin hardening associated with rheumatoid arthritis and autoimmune diseases were included, or whether microRNAs that controlled proteins and/or genes that were targets of therapeutic drugs were included. In this Test Example 2, a search was conducted for miRNAs targeting genes involved in the expression of PTX3 as target genes as proteins involved in vasculitis or skin hardening associated with rheumatoid arthritis and autoimmune diseases. Incidentally, for reference, a search was also conducted for miRNAs targeting genes involved in the expression of thrombospondin 1 (TSP1 or THBS1) as target genes.

The miRNAs expressed in exosomes purified from a culture supernatant of dental pulp-derived stem cells targeting the genes involved in the expression of PTX3 as targets, constituted the following PTX3 suppression-related miRNA group (3 types).
hsa-miR-224-5p, hsa-miR-374c-5p and hsa-miR-655-3p

Therefore, it was found that the expression controlling agent of the present invention can be used as a PTX3 expression controlling agent.

For reference, the miRNAs expressed in exosomes purified from the culture supernatant of dental pulp-derived stem cells targeting the genes involved in the expression of thrombospondin 1 (TSP1 or THBS1) as targets, constituted the following TSP1-related miRNA group (69 types).

### TSP1-related miRNA group:

hsa-let-7a-5p, hsa-let-7b-5p, hsa-let-7c-5p, hsa-let-7d-5p, hsa-let-7e-5p,
hsa-let-7f-5p, hsa-let-7g-5p, hsa-let-7i-5p, hsa-miR-101-3p, hsa-miR-125a-3p,
hsa-miR-1273g-3p, hsa-miR-132-3p, hsa-miR-139-5p, hsa-miR-141-3p, hsa-miR-143-3p,
hsa-miR-144-3p, hsa-miR-155-5p, hsa-miR-182-5p, hsa-miR-194-5p, hsa-miR-198,
hsa-miR-19a-3p, hsa-miR-19b-3p, hsa-miR-205-5p, hsa-miR-206, hsa-miR-211-3p,
hsa-miR-212-3p, hsa-miR-221-3p, hsa-miR-222-3p, hsa-miR-27b-3p, hsa-miR-297,
hsa-miR-300, hsa-miR-30a-3p, hsa-miR-30a-5p, hsa-miR-3149, hsa-miR-331-3p,
hsa-miR-338-3p, hsa-miR-340-5p, hsa-miR-3609, hsa-miR-3665, hsa-miR-377-3p,
hsa-miR-377-5p, hsa-miR-381-3p, hsa-miR-382-5p, hsa-miR-410-3p, hsa-miR-411-3p,
hsa-miR-4257, hsa-miR-4270, hsa-miR-4450, hsa-miR-4458, hsa-miR-4495,
hsa-miR-4500, hsa-miR-455-3p, hsa-miR-4655-5p, hsa-miR-4739, hsa-miR-491-5p,
hsa-miR-493-3p, hsa-miR-493-5p, hsa-miR-5003-5p, hsa-miR-509-5p, hsa-miR-5096, and
hsa-miR-543, hsa-miR-582-5p, hsa-miR-613, hsa-miR-650, hsa-miR-657,
hsa-miR-675-3p, hsa-miR-7-1-3p, hsa-miR-766-5p, hsa-miR-98-5p.

Therefore, it was found that the expression controlling agent of the present invention can be used as a thrombospondin 1 expression controlling agent.

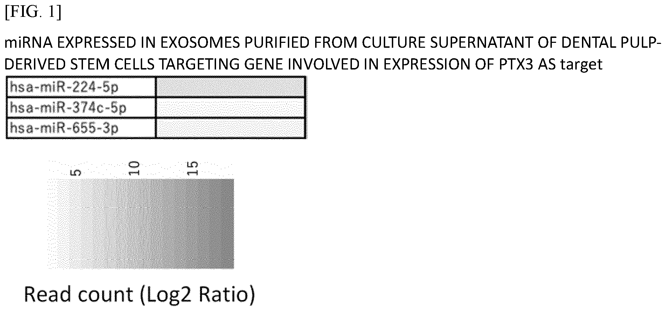

The results of comparing the expression levels of miRNAs expressed in the exosomes of dental pulp-derived stem cells are shown in the heat map of FIG. 1. The concentration in the heat map is expressed as the log ratio of read count values.

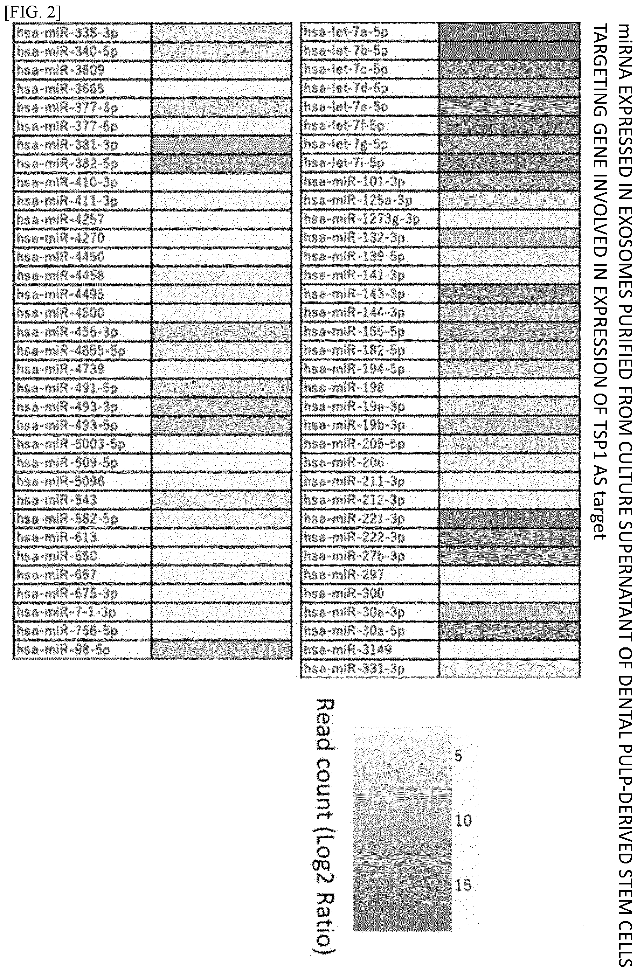

From FIG. 1 and FIG. 2, it was found that the expression controlling agent of the present invention contains a high concentration of miRNAs related to PTX3. Therefore, the expression controlling agent of the present invention can control the expression of PTX3 and/or PTX3 gene (Ptx3).

Incidentally, it was found that the expression controlling agent of the present invention contains a high concentration of miRNAs related to TSP-1. Therefore, the expression controlling agent of the present invention can control the expression of TSP-1 and/or TSP-1 gene (Tsp-1).

### [Test Example 3]: Verification of suppression of PTX3 expression

Suppression of PTX3 expression was checked by the following method, using a vasculitis model of human umbilical vein endothelial cells (HUVEC). Furthermore, for reference, the control of TSP-1 expression was also checked.

HUVEC cells were seeded and then cultured for 18 hours under the conditions of 37°C and 5% CO. Thereafter, the HUVEC cells were sealed in a BBL GasPak^{™} anaerobic system (Becton Dickinson Microbiology Systems, Cockeysville, MD, USA) and cultured under low oxygen conditions for 24 hours. As a result, the culture environment for the HUVEC cells has an oxygen concentration of 2%.

After 24 hours of the sealed culture, the expression controlling agent of Example 1 (exosomes purified from dental pulp-derived stem cells) or the small extracellular vesicle composition prepared in Comparative Example 1 (exosomes purified from umbilical cord-derived stem cells) was administered at a rate of 10000 exosomes per HUVEC cell.

After 48 hours, the expression level of PTX3 gene was quantitatively determined by qPCR using the following primer set.

### PTX3:

Pentraxin 3 (PTX3) Human qPCR Primer Pair (NM _002852)
CGAAATAGACAATGGACTCCATCC/CTCATCTGCGAGTTCTCCAGCA

Incidentally, for reference, the expression level of TSP-1 gene was quantitatively determined by qPCR using the following prime set, in the same manner as with PTX3 gene.

### TSP-1:

Thrombospondin 1 (THBS1) Human qPCR Primer Pair (NM_003246)
GCTGGAAATGTGGTGCTTGTCC/CTCCATTGTGGTTGAAGCAGGC

### [Reference Example 1]: Untreated with low oxygen

The expression level of PTX3 gene and the expression level of TSP-1 gene were quantitatively determined by qPCR in the same manner as in Example 1, except that the treatment of culturing HUVEC cells under low oxygen for 24 hours was replaced with culturing for 24 hours under the conditions of 37°C and 5% CO, and no small extracellular vesicle composition was added.

### [Reference Example 2]: Without small extracellular vesicles after low-oxygen culture

The expression level of PTX3 gene and the expression level of TSP-1 gene were quantitatively determined by qPCR in the same manner as in Example 1, except that no small extracellular vesicle composition was added.

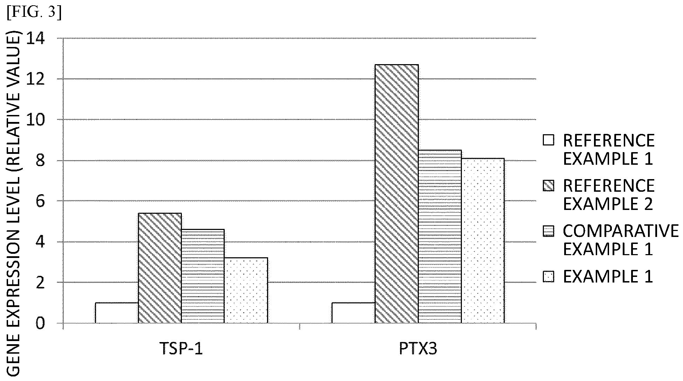

FIG. 3 is graphs of the expression level of PTX3 gene and the expression level of TSP-1 gene quantitatively determined in each example. From FIG. 3, it was found that the PTX3 expression controlling agent of the present invention can notably suppress the expression level of PTX3 gene.

Incidentally, it was found that the expression controlling agent of the present invention can also notably suppress the expression level of TSP-1 gene.

## Claims

1. A PTX3 expression controlling agent,
wherein the expression controlling agent comprises small extracellular vesicles, and
the small extracellular vesicles contain a miRNA targeting a gene involved in expression of PTX3 as a target gene.

2. The PTX3 expression controlling agent according to claim 1, wherein the small extracellular vesicles are exosomes.

3. The PTX3 expression controlling agent according to claim 1, wherein the expression controlling agent includes at least one type of hsa-miR-224-5p, hsa-miR-374c-5p, and hsa-miR-655-3p as the miRNA targeting a gene involved in the expression of PTX3 as a target gene.

4. The PTX3 expression controlling agent according to claim 1, wherein the miRNA targeting a gene involved in the expression of PTX3 as a target gene includes at least hsa-miR-224-5p.

5. The PTX3 expression controlling agent according to claim 1, wherein the miRNA targeting a gene involved in the expression of PTX3 as a target gene includes hsa-miR-224-5p and hsa-miR-655-3p.

6. The PTX3 expression controlling agent according to claim 1, wherein the small extracellular vesicles contain a miRNA targeting a gene involved in the expression of PTX3 as a target gene, at a concentration higher than that in a culture supernatant of dental pulp-derived stem cells.

7. The PTX3 expression controlling agent according to claim 1, wherein the small extracellular vesicles are small extracellular vesicles purified and isolated from a culture supernatant of dental pulp-derived stem cells, and the small extracellular vesicles do not contain any component other than exosomes from the culture supernatant of dental pulp-derived stem cells.

8. A prophylactic drug or therapeutic drug for vasculitis or skin hardening associated with rheumatoid arthritis and autoimmune diseases, the prophylactic drug or therapeutic drug comprising the PTX3 expression controlling agent according to claim 1 as an active ingredient.

9. The prophylactic drug or therapeutic drug for vasculitis or skin hardening associated with rheumatoid arthritis and autoimmune diseases according to claim 8, wherein the prophylactic drug or therapeutic drug is a prophylactic drug or therapeutic drug for skin hardening and is a skin application agent.

10. A method for improving vasculitis or skin hardening associated with rheumatoid arthritis and autoimmune diseases, the method comprising:
administering an effective amount of the PTX3 expression controlling agent according to claim 1 or an effective amount of the prophylactic drug or therapeutic drug for vasculitis or skin hardening associated with rheumatoid arthritis and autoimmune diseases according to claim 8, to a subject who has developed vasculitis or skin hardening associated with rheumatoid arthritis and autoimmune diseases.
